# EUROPEAN PATENT SPECIFICATION

(11) **EP 4 321 138 B1**
(45) Date of publication and mention of the grant of the patent: **28.08.2024**
(21) Application number: 22189516.2
(22) Date of filing: 09.08.2022
(51) Int. Cl.: A61F 13/494, A61F 13/496

(54) **ABSORBENT ARTICLE WITH TRANSVERSAL BARRIER**
SAUGFÄHIGER ARTIKEL MIT QUERBARRIERE
ARTICLE ABSORBANT DOTÉ D'UNE BARRIÈRE TRANSVERSALE

(43) Date of publication of application: 14.02.2024
(73) Proprietor: Ontex BV, 9255 Buggenhout (BE); Ontex Group NV, 9320 Erembodegem (BE)
(72) Inventor: HEEGE, Thomas, 56761 Düngenheim (DE); IFFLAND, Dirk, D-45529 Hattingen (DE); BUYSSE, Michiel, 9968 Oosteeklo (BE); PSCHYKLENK, Lukas, 56727 Müllenbach (DE); FRANSEN, Markus, 56269 Dierdorf (DE)
(74) Representative: Macchetta, Andrea

(56) References cited:
- WO-A1-2014/002440
- US-A1- 2012 022 492
- US-A1- 2018 338 870
- US-A1- 2021 205 148
- US-B1- 6 221 460
- US-B2- 10 159 611
- US-B2- 7 204 830

## Description

### TECHNICAL FIELD

The present disclosure is directed to an absorbent article for personal hygiene, typically in the form of pants (such as for babies, youths or adults) and generally of the disposable kind.

### BACKGROUND

Absorbent articles for personal hygiene are designed to absorb and contain bodily exudates, such as a large quantity of urine and/or stool. Non-limiting examples of disposable absorbent articles include diapers, pants, training pants, pADL, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp (also referred to as fluff pulp and/or cellulose fibers) with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

US10159611 B2 describes a multiple use absorbent article including a chassis absorbent unit having a chassis topsheet, a chassis backsheet, and a chassis absorbent core sandwiched between the chassis topsheet and the chassis backsheet. The article also includes an absorbent insert bonded to the chassis absorbent unit, a removable portion of the absorbent insert configured to be selectively removable from the chassis absorbent unit, wherein the absorbent insert includes an insert topsheet, an insert backsheet, and an insert absorbent core sandwiched between the insert topsheet and the insert backsheet. The absorbent insert further includes a line of weakness that provides for separation of the absorbent insert into a removable portion configured to be removed from the chassis absorbent unit, and a resident portion that remains bonded to the chassis absorbent unit.

WO2014/002440 A1 describes a diaper (1) including elastic front and rear waist panels (20, 30) respectively defining front and rear waist regions (12, 13), a crotch panel (40) defining a crotch region (14) and attached to the front and rear waist panels (20, 30) and an absorbent structure (50) located on an interior side of the crotch panel (40) and extending in a longitudinal direction (Y). A dimension (L1) in a longitudinal direction (Y) of the crotch panel (40) is smaller than a dimension (L2) of the absorbent structure (50) and front and rear end portions (50A, 50B) of the absorbent structure (50) lie outboard of front and rear end portions (40A, 40B) of the crotch panel (40) as viewed in a longitudinal direction (Y). A dimension (W1) in a transverse direction (X) of the crotch panel (40) is larger than a dimension (W2) of the absorbent structure (50) and lateral edge portions (40C) of the crotch region (40) extend outwardly in the transverse direction (X) beyond lateral edge portions (50C) of the absorbent structure (50).

A number of disclosures exist (see for example EP3451989 B1) directed to limiting the risk of leakage by providing transversal barriers at the front and/or back of a diaper. However whilst such executions may be effective on diaper constructs they are less effective and or complex/costly to incorporate into a pant concept.

An attempt to overcome such shortcomings has been made in for example WO2021/170027 A1 wherein a nonwoven layer of the front and/or back belt is folded a plurality of times to form a waist guard. Although this provides for an effective way to introduce a transversal barrier in a pant construct, there are several drawbacks that have been identified: firstly, the nonwoven that is generally hydrophobic may be sufficient to capture solids but is less effective to capture larger amounts of liquid exudates (particularly under pressure such as when a baby is moving and/or application of sudden pressure such as sitting/dropping on the floor on its buttocks); secondly it limits the positioning thereof to a closer position at the waist edge which is less effective in providing a barrier since it is positioned further away from the core and to position it closer to the core and away from the waist edge would require a significant increase in length of the nonwoven which adds significant waste and cost; thirdly applying the multiple folds at different positions of the same component and especially further elasticizing the region when/if desired may add significant process complexity particularly at high speeds.

A need therefore exists for improved absorbent articles having leakage protection particularly for substantially liquid exudates such as urine and/or liquid stool yet in in a cost effective manner and with more limited additional waste.

### SUMMARY

In a first aspect the disclosure relates to an absorbent article for personal hygiene, preferably a disposable pant, comprising: a front panel preferably substantially transversely extending; a back panel preferably substantially transversely extending; and an absorbent insert, preferably substantially longitudinally extending, joined to each of said front and back panels, said absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween). The front and back panels are joined together to define a pair of side seams, and at least a portion of said absorbent insert proximal to the front and/or back transversal edges thereof comprises a transversal waist barrier comprising a substantially liquid impermeable film layer.

In a second aspect the disclosure relates to a method for the manufacture of an absorbent article comprising the steps of: providing a front panel; providing a back panel; providing an absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); folding a portion of said absorbent insert proximal to the front and/or back transversal edges thereof onto itself, preferably in the shape of a substantially U-fold, to form transversal waist barrier(s); joining said insert to said front and back panels; and joining said front and back panels together along a pair of oppositely disposed side seams.

### BRIEF DESCRIPTION OF THE FIGURES

**FIG. 1** is a top planar view of an absorbent article according to an embodiment of the present disclosure.
**FIG. 2** is a top planar view of an absorbent article according to an embodiment of the present disclosure.
**FIG. 3** is a partial cross-section of an absorbent article according to an embodiment of the present disclosure.
**FIG. 4** is a top planar view of an absorbent article according to an embodiment of the present disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

The values indicated herein are measured according to the methods indicated herein below, unless specified otherwise. All measurements are performed at 21±2° C. and 50±20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### THE ABSORBENT ARTICLE

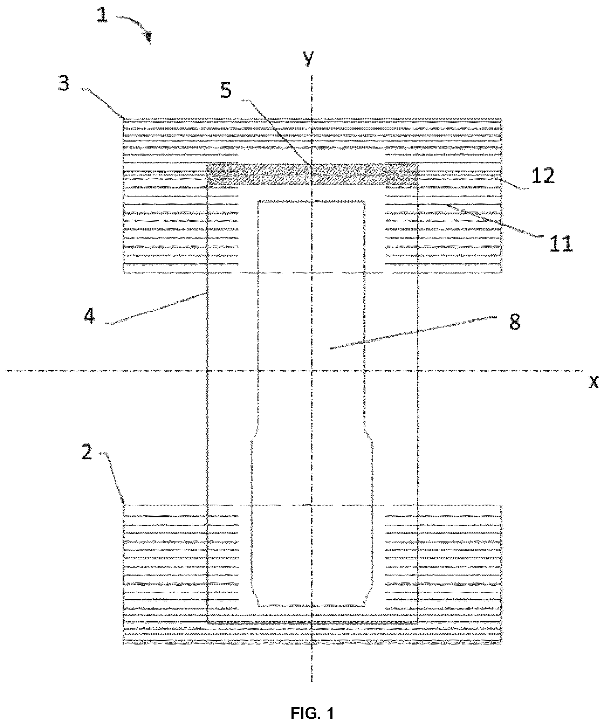

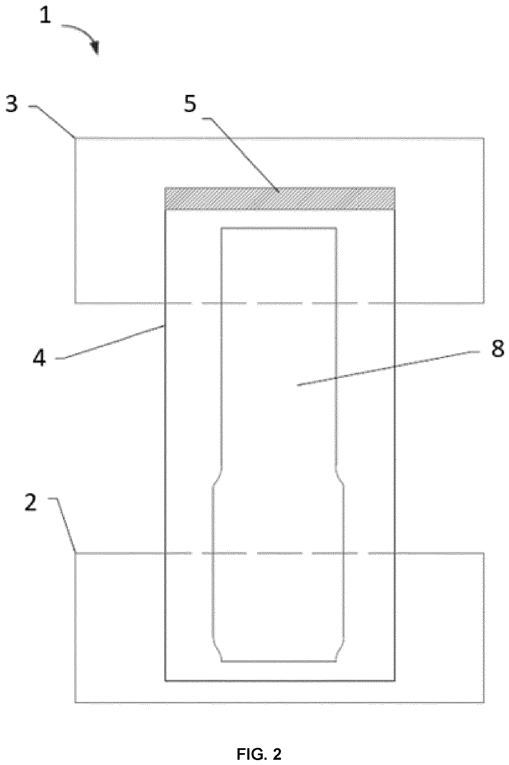

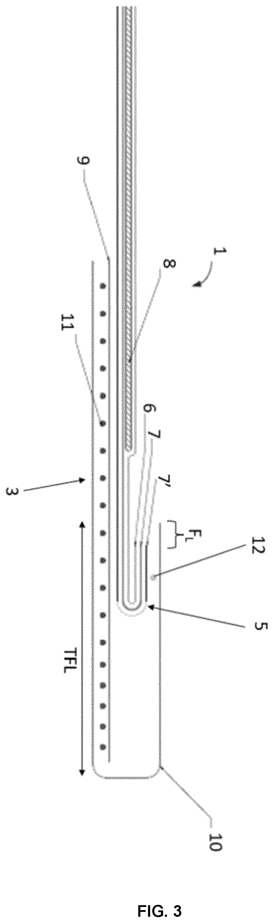

As exemplified in Fig.1 to Fig.3, absorbent articles herein are for personal hygiene, preferably in the form of a pant, the absorbent article comprising: a substantially transversely extending front panel (2); a substantially transversely extending back panel (3); and a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof (generally wherein the left and right longitudinal edges extend substantially parallel to the longitudinal axis y and are oppositely disposed such that said longitudinal axis y extends therebetween, and the front and back transversal edges extend substantially parallel to the transversal axis x, and typically substantially perpendicular to the longitudinal axis y, and are oppositely disposed such that said transverse axis x extends therebetween); wherein the front and back panels (2, 3) are joined together to define a pair of side seams, and wherein at least a portion of said absorbent insert (4) proximal to the front and/or back transversal edges thereof comprises a transversal waist barrier(s) (5) comprising a substantially liquid impermeable film layer, preferably wherein said transversal waist barrier (5) is an up-standing barrier generally arranged to block substantially liquid exudates from flowing therethrough. Advantageously, this arrangement allows for an absorbent article with superior leakage prevention particularly for substantially liquid exudates such as urine and/or liquid stool.

Generally, the substantially liquid impermeable film layer is an integral component of at least one element or portion of the absorbent article such as a backsheet (7) thereof typically such that no additional and/or separate layer, element and/or material is incorporated to form the transversal waist barrier(s) (5). Advantageously this allows to reduce waste and/or cost associated with incorporating separate elements to create the transversal waist barrier(s).

The backsheet (7) is generally that portion of the absorbent article positioned adjacent the garment-facing surface of the absorbent core (8) and which prevents the exudates absorbed and contained therein from soiling articles such as bedsheets and undergarments. The backsheet (7) is typically impermeable to liquids (e.g. urine). The backsheet (7) may for example be or comprise a thin plastic film such as a thermoplastic film having a thickness of about 0.012 mm to about 0.051 mm. Preferably the basis weight of the backsheet (7) is less than or equal to 16 g/m², more preferably from 10 g/m² to 14 g/m². Exemplary backsheet films include those manufactured by Tredegar Corporation, based in Richmond, Va., and sold under the trade name CPC2 film. Other suitable backsheet materials may include breathable materials which permit vapors to escape from the absorbent article while still preventing exudates from passing through the backsheet (7). Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Some breathable composite materials are described in greater detail in PCT Application No. WO 95/16746 published on Jun. 22, 1995 in the name of E. I. DuPont; U.S. Pat. No. 5,938,648 to LaVon et al., U.S. Pat. No. 4,681,793 to Linman et al., U.S. Pat. No. 5,865,823 to Curro; and U.S. Pat. No. 5,571,096 to Dobrin et al, U.S. Pat. No. 6,946,585B2 to London Brown.

In a preferred embodiment the backsheet (7) is breathable. Breathable backsheet herein typically means that it comprises micro-openings sized to allow at least some water vapour to permeate through the backsheet that typically comprises a film such as a polyethylene (PE) film.

Preferably a backsheet (7) is breathable when the water vapour transmission rate (WVTR) of the backsheet is at least 500 grams/m2 - 24 hours, preferably at least 1,000 grams/m2 - 24 hours, even more preferably from 1,200 grams/m2 - 24 hours to 2,500 grams/m2 - 24 hours, even more preferably from 1,500 grams/m2 - 24 hours to 2,100 grams/m2 - 24 hours, as measured according to ASTM D6701-21.

The backsheet (7) may be joined to the topsheet (6), the absorbent core (8) or any other element of the absorbent article by any attachment means known in the art. Suitable attachment means are described above with respect to means for joining the topsheet to other elements of the article. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core (8) of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

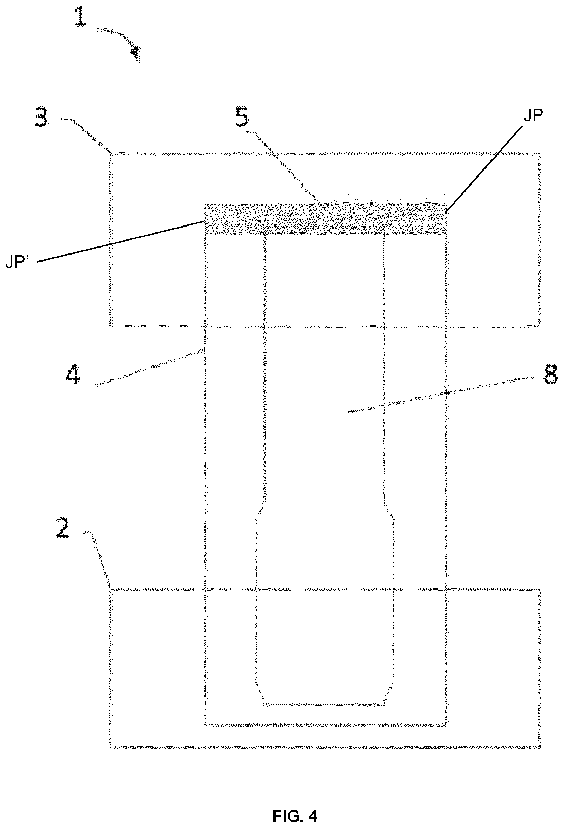

In an embodiment, as exemplified in Fig. 4, the transversal waist barrier(s) (5) overlap at least a portion of an absorbent core (8) of the insert (4) typically such that the exudates collected and/or retained by the transversal waist barrier(s) (5) are substantially dehydrated and/or absorbed by the neighbouring absorbent core (8) surface(s). Advantageously this allows for reduced leakage risks of highly liquid exudates like urine or liquid stool such as diarrhoea (or other liquid stool that is generally and commonly observed e.g. with newborn babies).

Preferably the front and back panels (2, 3) are separate and connected to each other by the insert (4) acting as a bridge element to form a substantially H-shaped pant. Advantageously this allows to omit cutting/shaping of panels in a crotch region of the article thus reducing waste and/or limiting the amount of panel material in view of its absence in the crotch region also helping to reduce waste and cost.

The insert (4) may be joined to the front and back panels (2, 3) by adhesive and/or mechanical bonding such as ultrasonic bonding, pressure bonding, and/or thermal bonding and the like. In a preferred embodiment the insert (4) is joined to the front and back panels (2, 3) by a discontinuous pattern of adhesive. Advantageously this may help to retain softness and pliability of the laminated structures and reduce stiffness when worn.

In an embodiment, the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene. Advantageously such transversal waist barrier(s) are not only impermeable to a much higher degree than hydrophobic nonwovens but further are formed by using a component already present in the absorbent article and hence does not require adding any further materials that would impact waste and/or cost.

Preferably, the at least portion of said absorbent insert (4) proximal to the front and/or back transversal edges thereof comprises one or more folds forming the transversal waist the absorbent article and hence does not require adding any further materials that would impact waste and/or cost.

The at least portion of said absorbent insert (4) proximal to the front and/or back transversal edges thereof comprises one or more folds forming the transversal waist barrier(s) (5), preferably wherein said fold is substantially U-shaped. Advantageously such fold of the insert allows to form a liquid impermeable barrier to exudates, and particularly the U-shape (vs other shapes) allows to form a maximised pocket for exudate collection with the minimum use of material.

Preferably, the folded portion of said absorbent insert (4) is joined to the body-facing surface of the topsheet (6) at a pair of oppositely disposed extremities of the insert (4) along a portion of the left and right longitudinal edges thereof, generally wherein said oppositely disposed extremities are connected by a fold of said folded portion of said absorbent insert (4), such that a pocket is formed that acts as a barrier to exudates in both the longitudinal direction (i.e. substantially parallel to the longitudinal axis y) and the transverse direction (i.e. substantially parallel to the transverse axis x). The said extremities of the insert (4) may be joined by adhesive and/or mechanical bonding such as ultrasonic bonding, thermal bonding, and/or pressure bonding and the like.

In an embodiment, the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction (as generally depicted and illustrated in the figures herein and that typically corresponds to a plane formed by the x and y axis described and illustrated herein) such that the one or more folds are substantially free of absorbent material. Advantageously, a fold that is substantially free of absorbent material allows for a more flexible fold that can allow the barrier to better stand upright upon application of tension and yet limit piercing of the topsheet and/or backsheet that could arise if exerting a folding force in an area of the insert comprising SAP particles.

In an embodiment, the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the backsheet with nonwoven so that the backsheet does not come into contact with the skin of a wearer which could otherwise result in skin irritations and/or less comfort.

The inner and outer nonwoven layers (9, 10) may be the same or different meaning that they may have the same or different properties selected from basis weight (in gsm) and composition. In an embodiment, both the inner and outer nonwoven layers (9, 10) each comprise a spunbond nonwoven, typically comprising monocomponent fibers of polypropylene, and each have a basis weight of from 10 g/m² (gsm) to 30 g/m² (gsm), preferably from 12 g/m² (gsm) to 25 g/m². The outer nonwoven layer (10) may have a basis weight that is higher than the inner nonwoven layer (9). Advantageously, although it is customary to have the higher basis weight nonwoven on the inner nonwoven wearer skin facing side, in barrier embodiments herein (especially when the outer nonwoven layer is folded and/or extends over the insert; and/or when the transversal barrier is elasticised) such arrangement provides for added mechanical integrity desirable for resistance to tear on elastification and/or added softness for the wearer on the front and/or back waist regions up to the barrier location.

Preferably, and as exemplified in Fig. 1, the elastic material (11), such as the plurality of elastic strands, is/are deactivated in an area of overlap of the insert (4) when viewed in a planar direction. The deactivation is preferably achieved by cutting of the elastic strands such as to render the deactivated region of the panel(s) (2, 3) substantially inelastic and regions neighbouring said deactivated region and comprising said elastic strands being elastic. Typically the absorbent core (8) of the insert (4) is positioned within the deactivated region such that it substantially does not overlap the elastic strands (11). Advantageously this allows for improved core integrity as risks of core collapse due to excessive tension from the stretched elastics when the article is worn is limited.

Preferably more than 55%, preferably more than 60%, even more preferably from 65% to 95%, of the total surface area (generally when viewed in a planar direction) of the transversal waist barrier(s) (5) is positioned to overlap the deactivated region of the panel(s) (2, 3). Advantageously this allows for improved fit of the barrier when the article is worn by a subject.

In addition or alternatively, more than 50%, preferably more than 60%, even more preferably from 65% to 95%, of a total transversal length (generally substantially parallel to the transversal axis x) of the transversal waist barrier(s) (5) overlaps the deactivated region of the panel(s) (2, 3). Especially when the transversal waist barrier(s) is elastic, this arrangement may advantageously not only improve core integrity but further avoid excessive tension build-up.

In an embodiment, the transversal waist barrier(s) (5) is elastic, preferably comprising an elastic material selected from the group consisting of an elastic film and one or more elastic strands. Advantageously this allows for application of tension that permits the barrier to stand upright in close contact to the skin when the article is worn and under stretch.

The elastic strands in any of the embodiments herein may be strand-coated with adhesive prior to joining to one or more layers such as the inner and outer nonwoven layers (9, 10) to form elastic panels (2, 3) and/or the backsheet (7), outer cover (7'), and/or outer nonwoven layer (10) to form the elastic waist barrier(s) (5). Advantageously this allows to join the one or more layers together by the said strand-coated elastic strands and by doing so limit the amount of adhesive used with resulting improved softness as well as reduced waste. Alternatively, or additionally, adhesive in the form of a plurality of stripes is applied such as by slot coating.

In a preferred embodiment, the one or more elastic strands of the transversal waist barrier(s) (5) comprise at least one flat elastic (such as elastics having a cross-section with an aspect ratio of longest to smallest dimension of greater than 1, preferably greater than 1.5, even more preferably greater than 2), preferably in combination with at least one or at least two round elastics (such as elastics having a cross-section with an aspect ratio of longest to smallest dimension of about 1). Although flat elastics are generally more expensive they are advantageous in providing better gasketing effects and comfort on the wearer's skin, by combining flat and normal/round elastics one can achieve the desired gasketing and comfort whist limiting the cost.

When one or more, preferably only one, flat elastic is used, it is preferably positioned adjacent to an apex of the transversal waist barrier(s) (5) that may be closest to a transversal edge of the insert (4) and further round elastic(s) positioned further from said apex compared to the flat elastic(s). Advantageously this allows for the flat elastic to be positioned at the closest contact position to the wearer with other elastics being inboard (or further away) therefrom hence contributing to the desired tensile strength yet permitting to lower the overall density/dtex of the flat elastic and hence also cost, with limited impact on performance.

When elastic strands are used they may have a dtex in the range of from 350 to 1100, preferably from 400 to 1000, even more preferably from 450 to 900. When elastic strands are used in all of the front and/or back panels (2, 3) and the waist barrier(s) (5), the elastic strands of said panel(s) (2, 3) have a dtex that is equal to or greater than, preferably greater than, that of the elastic strand(s) of said transversal waist barrier(s) (5). Preferably the elastic strand(s) of said transversal waist barrier(s) (5) have a dtex that is from 40% to 85%, preferably from 45% to 75%, even more preferably from 50% to 70%, of the dtex of the elastic strands of said panel(s) (2, 3). Advantageously, this allows the barrier(s) to stand up and provide a gasketing effect close to the skin of the wearer without flattening or adding excessive resistance to stretch that would otherwise impact fit and comfort. The tension differential that results entails that a lower tension in the barrier(s) compared to the belt(s) is generated to a degree that upon stretching of the belt(s) the barrier(s) stand-up away from said belt(s).

The tensile stress (N/m) of the entirety of the front and back panels (2, 3), respectively, may be profiled in order to provide the functional benefits of the present disclosure, such as ease of stretch and application, while also maintaining certain force during wear, to prevent the article from sagging after loading. When the elasticity of the front and back panels (2, 3) are provided by a plurality of elastic strands (11) running in the transverse direction, the tensile stress may be adjusted by one or more of the following methods; 1) elongation rate of the elastic strands (11); 2) density (dtex) of the elastic strands (11); 3) longitudinal pitch of multiple elastic strands (11); and 4) effective length of elasticity of the elastic strands (11) in the transverse direction. By elongation, "0% elongation" is meant the original length of the elastic member.

The front and back panels (2, 3) may each be divided into multiple zones spanning in the transverse direction and defined by its location from the distal edge to the proximal edge relative to the percentage of the seam length wherein the distal edge is considered 0% and the proximal edge is considered 100%. The multiple zones may be configured to provide different tensile stress, or different functions to the front and back panels (2, 3), respectively.

In an embodiment, one or more second elastic strands (12) are comprised directly or indirectly between the outer nonwoven layer (10) and the backsheet (7), preferably between a body-facing surface of the outer nonwoven layer (10) and a garment-facing surface of the backsheet (7). An example of an indirect elastic arrangement is the presence of an outer cover layer (7') as described herein (that would generally be interposed between the backsheet (7) and the one or more second elastic strands (12)), and an example of a direct elastic arrangement is the absence of an outer cover layer (7') as described herein. Advantageously this arrangement allows for a simple yet effective elastification of the barrier(s) permitting optimal gasketing effects.

Preferably, the one or more second elastic strands (12) extends along the entire transversal length of the transversal waist barrier(s) (5) and beyond, preferably crossing the imaginary longitudinal axis (y) in a direction substantially parallel to the imaginary transverse axis (x). Preferably the one or more second elastic strands (12) extends from a first position substantially adjacent a seam edge of the outer nonwoven layer (10) to a second position substantially adjacent an opposite seam edge of the outer nonwoven layer (10) with the imaginary longitudinal axis (y) being positioned therebetween, more preferably wherein the one or more second elastic strands (12) extends from said first position to said second position and crosses and/or overlaps the entire transversal waist barrier(s) (5) generally along the imaginary transverse axis (x). Advantageously this allows not only to form a standing barrier upon stretching but further aids better fit on the wearer's front and/or back waist.

In an embodiment, the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein. The superabsorbent polymer particles and/or fibers may be comprised in an amount of greater than 50%wt, preferably greater than 60%wt, even more preferably greater than 70%wt, most preferably from 75% to 100%wt, by total weight of absorbent material.

In an embodiment, the back panel (3) is wider (generally in a direction substantially parallel to the longitudinal axis y) than the front panel (2) such that a length of the seams is substantially equal to a width (generally in a direction substantially parallel to the longitudinal axis y) of the front panel (2) and less than a width of the back panel (3); or wherein the front panel (2) is wider than the back panel (3) such that a length of the seams is substantially equal to a width of the back panel (3) and less than a width of the front panel (2).

Advantageously this may permit comfort as well as improved protection compared to symmetric arrangements.

It is preferred that the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction (generally parallel to the longitudinal axis y) than the topsheet (6) and/or backsheet (7) such that at least a portion of an overlap length between the insert (4) and the panel (2, 3) is free of said outer cover layer (7') and preferably wherein the backsheet (7) is directly joined to, or is in contact with, the panel (2, 3) in said portion of an overlap length between the insert (4) and the panel (2, 3) free of said outer cover layer (7'). Advantageously this allows for improved softness in areas where needed and yet allow for material savings thus permitting both reduced cost and waste.

The outer cover layer (7') is preferably different from the inner and/or outer nonwoven layers (9, 10) meaning that they may have different properties selected from basis weight (in gsm) and composition. In an embodiment, the outer cover (7') comprises a spunbond nonwoven preferably comprising bicomponent fibers typically selected from the group consisting of polypropylene and polyethylene. The outer cover (7') preferably has a basis weight that is less than the basis weight of the inner and/or outer nonwoven layers (9, 10). The outer cover (7') may have a basis weight of from 8 g/m² (gsm) to 20 g/m² (gsm), preferably from 10 g/m² (gsm) to 15 g/m². Advantageously, the outer cover provides for softness to the touch to the care giver yet, as selected, it limits stress build-up particularly in embodiments with reduced length in the longitudinal direction as described above. Indeed without wishing to be bound by theory, stress build-up in the transition region corresponding to the terminal edge of the outer cover may result in a higher risk of tearing in the insert to panel joint.

In a preferred embodiment, the absorbent insert (4) is folded-over to form the waist barrier(s) (5) such that at least a portion of the topsheet (6) of the non-folded part of said insert (4) may come into contact with the folded part of said insert (4). Advantageously this allows the barrier to overlap the topsheet so as to help guide the exudates therethrough and into the core once stopped by said barrier.

Preferably, the waist barrier(s) (5) extends from 5% to 50%, preferably from 10% to 40%, even more preferably from 15% to 30%, of the total folded length (TFL) of the outer nonwoven layer (10) along the longitudinal axis (y). Advantageously, this may allow formation of a sufficiently large pocket yet avoid excessive resizing of the insert that may add cost and waste.

In an embodiment, the substantially liquid impermeable film layer or backsheet (7) comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive (preferably comprising one or more pigments). Advantageously this allows the user or care giver to better visualise the barrier(s) so as to aid correct positioning and placement thereof onto a subject.

In an embodiment, the absorbent article further comprising a pair of longitudinally extending cuffs positioned along left and right longitudinal edges of the insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (JP) corresponding to the transversal waist barrier(s) (5), preferably said joints or joining positions (JP) extending along a lengthwise portion of, and being substantially adjacent to, the left and/or right longitudinal edges of the insert (4). Advantageously this reduces risk of leakage multidirectionally and prevents leakage to seep through between the transversal barrier and cuffs unlike when otherwise arranged.

### THE METHOD

Methods herein for the manufacture of an absorbent article (1) comprise the steps of: providing a front panel (2); providing a back panel (3); providing an absorbent insert (4) comprising front and back transversal edges and left and right longitudinal edges connecting the front and back transversal edges to form a perimeter thereof (and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); folding a portion of said absorbent insert (4) proximal to the front and/or back transversal edges thereof onto itself, preferably the fold being in the shape of a substantially U-fold, to form transversal waist barrier(s) (5); joining said insert (4) to said front and back panels (2, 3); and joining said front and back panels (2, 3) together along a pair of oppositely disposed side seams. Advantageously an absorbent article having transversal barrier(s) as described herein can be attained in a simple yet effective manner.

Preferably, the method further comprises the steps of folding an outer nonwoven layer (10) of the front and/or back panels (2, 3) over the folded absorbent insert (4) and/or an inner nonwoven layer (9) and joining said folded outer nonwoven layer (10) directly or indirectly to the folded absorbent insert (4). Advantageously this allows to prevent irritation or discomfort to the wearer as explained hereinabove whilst avoiding the use of added components/materials/elements whilst maintaining a simple and cost-effective production process.

## Claims

1. An absorbent article (1) for personal hygiene, preferably a disposable pant, comprising:
a substantially transversely extending front panel (2);
a substantially transversely extending back panel (3); and
a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof;
wherein the front and back panels (2, 3) are joined together to define a pair of side seams, and **characterised in that** at least a portion of said absorbent insert (4) proximal to the front and/or back transversal edges thereof comprises a transversal waist barrier (5) comprising a substantially liquid impermeable film layer, and wherein said at least portion of said absorbent insert (4) proximal to the front and/or back transversal edges thereof comprises one or more folds forming the transversal waist barrier(s) (5).

2. An absorbent article (1) according to Claim 1 wherein the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene.

3. An absorbent article (1) according to any of the preceding Claims wherein said fold is substantially U-shaped.

4. An absorbent article (1) according to any of the preceding Claims wherein the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction such that the one or more folds are substantially free of absorbent material.

5. An absorbent article (1) according to any of the preceding Claims wherein the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands.

6. An absorbent article (1) according to Claim 5 wherein the outer nonwoven layer (10) is wider than the inner nonwoven layer (9), generally in a direction substantially parallel to the longitudinal axis y, and is folded over said inner nonwoven layer (9) such that it overlaps the portion of said absorbent insert (4) proximal to the front and/or back transversal edges, preferably such that said outer nonwoven layer (10) overlaps both a portion of the backsheet (7) and a portion of the topsheet (6) that remains exposed from said one or more transversal waist barrier(s) (5) such to form a flange (F_{L}).

7. An absorbent article (1) according to any of the preceding Claims wherein the transversal waist barrier(s) (5) is elastic, preferably comprising an elastic material selected from the group consisting of an elastic film and one or more elastic strands.

8. An absorbent article (1) according to Claims 5 to 7 wherein one or more second elastic strands (12) are comprised directly or indirectly between the outer nonwoven layer (10) and the backsheet (7), preferably between a body-facing surface of the outer nonwoven layer (10) and a garment-facing surface of the backsheet (7).

9. An absorbent article (1) according to any of the preceding Claims wherein the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein.

10. An absorbent article (1) according to any of the preceding Claims wherein the back panel (3) is wider than the front panel (2) such that a length of the seams is substantially equal to a width of the front panel (2) and less than a width of the back panel (3); or wherein the front panel (2) is wider than the back panel (3) such that a length of the seams is substantially equal to a width of the back panel (3) and less than a width of the front panel (2).

11. An absorbent article (1) according to Claims 2 to 10 wherein the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction than the topsheet (6) and/or backsheet (7) such that at least a portion of an overlap length between the insert (4) and the panel (2, 3) is free of said outer cover layer (7') and preferably wherein the backsheet (7) is directly joined to, or in contact with, the panel (2, 3) in said portion of an overlap length between the insert (4) and the panel (2, 3) free of said outer cover layer (7').

12. An absorbent article (1) according to any of the preceding Claims wherein the absorbent insert (4) is folded-over to form the waist barrier(s) (5) such that the topsheet (6) of the non-folded part of said insert (4) is able to come into contact with the folded part of said insert (4).

13. An absorbent article (1) according to Claims 6 to 12 wherein the waist barrier(s) (5) extends from 5% to 50%, preferably from 10% to 40%, even more preferably from 15% to 30%, of the total folded length (TFL) of the outer nonwoven layer (10) along the longitudinal axis (y).

14. An absorbent article (1) according to any of the preceding Claims wherein the substantially liquid impermeable film layer, or backsheet (7), comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive.

15. An absorbent article (1) according to any of the preceding Claims further comprising a pair of longitudinally extending cuffs positioned along left and right longitudinal edges of the insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (J P) substantially corresponding to the transversal waist barrier(s) (5) to form an exudate collection pocket.

16. A method for the manufacture of an absorbent article (1) comprising the steps of:
providing a front panel (2);
providing a back panel (3);
providing an absorbent insert (4) comprising front and back transversal edges and left and right longitudinal edges connecting the front and back transversal edges to form a perimeter thereof;
folding a portion of said absorbent insert (4) proximal to the front and/or back transversal edges thereof onto itself to form transversal waist barrier(s) (5);
joining said insert (4) to said front and back panels (2, 3);
and joining said front and back panels (2, 3) together along a pair of oppositely disposed side seams.

17. A method according to Claim 16 further comprising the steps of folding an outer nonwoven layer (10) of the front and/or back panels (2, 3) over the folded absorbent insert (4) and joining said folded outer nonwoven layer (10) directly or indirectly to the folded absorbent insert (4).

## Patentansprüche

1. Saugfähiger Artikel (1) für die Intimhygiene, vorzugsweise ein Einweghöschen, umfassend:
eine sich im Wesentlichen quer erstreckende Vorderseite (2);
eine sich im Wesentlichen quer erstreckende Rückseite (3); und
eine sich im Wesentlichen längs erstreckende saugfähige Einlage (4), die mit jeder von der Vorder- und Rückseite (2, 3) verbunden ist, wobei die saugfähige Einlage (4) vordere und hintere Querkanten und linke und rechte Längskanten umfasst, welche die vorderen und hinteren Querkanten verbinden, um einen Umfang davon zu bilden;
wobei die Vorder- und Rückseite (2, 3) miteinander verbunden sind, um ein Paar Seitennähte zu definieren, und **dadurch gekennzeichnet, dass** mindestens ein Abschnitt der saugfähigen Einlage (4) proximal zu den vorderen und/oder hinteren Querkanten davon eine quer verlaufende Taillenbarriere (5) umfasst, die eine im Wesentlichen flüssigkeitsundurchlässige Folienschicht umfasst, und wobei der mindestens Abschnitt der saugfähigen Einlage (4) proximal zu den vorderen und/oder hinteren Querkanten davon eine oder mehrere Falten umfasst, welche die quer verlaufende(n) Taillenbarriere(n) (5) bilden.

2. Saugfähiger Artikel (1) nach Anspruch 1, wobei die im Wesentlichen flüssigkeitsundurchlässige Folienschicht eine Rücklage (7) der saugfähigen Einlage (4) umfasst, vorzugsweise daraus besteht, und vorzugsweise ein Material umfasst, das Polyester umfasst oder daraus besteht, wobei das Material bevorzugter aus der Gruppe ausgewählt ist, die aus Polyethylen, Polymilchsäure und Polypropylen besteht.

3. Saugfähiger Artikel (1) nach einem der vorstehenden Ansprüche, wobei die Falte im Wesentlichen U-förmig ist.

4. Saugfähiger Artikel (1) nach einem der vorstehenden Ansprüche, wobei die saugfähige Einlage (4) eine im Wesentlichen flüssigkeitsdurchlässige Oberlage (6), eine im Wesentlichen flüssigkeitsundurchlässige Rücklage (7) und einen dazwischen eingelegten saugfähigen Kern (8) aufweist, wobei der saugfähige Kern (8) bei Betrachtung in einer planaren Richtung vorzugsweise innerhalb des Umfangs der saugfähigen Einlage (4) positioniert ist, sodass die eine oder mehreren Falten im Wesentlichen frei von saugfähigem Material sind.

5. Saugfähiger Artikel (1) nach einem der vorstehenden Ansprüche, wobei die Vorder- und/oder Rückseite (2, 3) elastisch sind und eine innere Vliesschicht (9), eine äußere Vliesschicht (10) und ein dazwischen eingelegtes elastisches Material (11) umfassen, wobei das elastische Material aus der Gruppe ausgewählt ist, die aus einer elastischen Folie und einer Vielzahl von elastischen Fäden besteht.

6. Saugfähiger Artikel (1) nach Anspruch 5, wobei die äußere Vliesschicht (10) breiter ist als die innere Vliesschicht (9), im Allgemeinen in einer Richtung im Wesentlichen parallel zur Längsachse y, und über die innere Vliesschicht (9) gefaltet ist, sodass sie den Abschnitt der saugfähigen Einlage (4) proximal zu den vorderen und/oder hinteren Querkanten vorzugsweise so überlappt, dass die äußere Vliesschicht (10) sowohl einen Abschnitt der Rücklage (7) als auch einen Abschnitt der Oberlage (6) überlappt, der von der (den) einen oder mehreren quer verlaufenden Taillenbarriere(n) (5) freigelegt bleibt, um einen Flansch (F_{L}) zu bilden.

7. Saugfähiger Artikel (1) nach einem der vorstehenden Ansprüche, wobei die quer verlaufende(n) Taillenbarriere(n) (5) elastisch ist/sind, vorzugsweise ein elastisches Material umfasst/umfassen, das aus der Gruppe ausgewählt ist, die aus einer elastischen Folie und einem oder mehreren elastischen Fäden besteht.

8. Saugfähiger Artikel (1) nach den Ansprüchen 5 bis 7, wobei ein oder mehrere zweite elastische Fäden (12) direkt oder indirekt zwischen der äußeren Vliesschicht (10) und der Rücklage (7), vorzugsweise zwischen einer dem Körper zugewandten Oberfläche der äußeren Vliesschicht (10) und einer dem Kleidungsstück zugewandten Oberfläche der Rücklage (7), umfasst sind.

9. Saugfähiger Artikel (1) nach einem der vorstehenden Ansprüche, wobei der saugfähige Kern (8) saugfähiges Material umfasst, das aus der Gruppe ausgewählt ist, die aus super saugfähigen Polymerteilchen und/oder -fasern; und Zellulosefasern besteht, wobei das saugfähige Material vorzugsweise innerhalb einer Kernumwicklung eingeschlossen ist, die obere und untere Kernumwicklungsschichten umfasst, die eine gleiche Vliesschicht sind, die gefaltet ist, um das saugfähige Material darin einzulegen, oder verschiedene Vliesschichten, die miteinander verbunden sind, um das saugfähige Material darin einzulegen.

10. Saugfähiger Artikel (1) nach einem der vorstehenden Ansprüche, wobei die Rückseite (3) breiter als die Vorderseite (2) ist, sodass eine Länge der Nähte im Wesentlichen gleich einer Breite der Vorderseite (2) und kleiner als eine Breite der Rückseite (3) ist; oder wobei die Vorderseite (2) breiter als die Rückseite (3) ist, sodass eine Länge der Nähte im Wesentlichen gleich einer Breite der Rückseite (3) und kleiner als eine Breite der Vorderseite (2) ist.

11. Saugfähiger Artikel (1) nach den Ansprüchen 2 bis 10, wobei die Rücklage (7) eine weitere äußere Deckschicht (7') auf einer dem Kleidungsstück zugewandten Oberfläche davon umfasst, wobei die äußere Deckschicht (7') vorzugsweise eine oder mehrere Vliesschichten umfasst, und/oder wobei die Rücklage (7) eine weitere äußere Deckschicht (7') umfasst, die in der Längsrichtung kürzer ist als die Oberlage (6) und/oder die Rücklage (7), sodass mindestens ein Abschnitt einer Überlappungslänge zwischen der Einlage (4) und der Seite (2, 3) frei von der äußeren Deckschicht (7') ist, und wobei die Rücklage (7) vorzugsweise direkt mit der Seite (2, 3) in dem Abschnitt einer Überlappungslänge zwischen der Einlage (4) und der Seite (2, 3), die frei von der äußeren Deckschicht (7') ist, verbunden ist oder damit in Kontakt steht.

12. Saugfähiger Artikel (1) nach einem der vorstehenden Ansprüche, wobei die saugfähige Einlage (4) umgefaltet ist, um Taillenbarriere(n) (5) zu bilden, sodass die Oberlage (6) des nicht gefalteten Teils der Einlage (4) in der Lage ist, mit dem gefalteten Teil der Einlage (4) in Kontakt zu kommen.

13. Saugfähiger Artikel (1) nach den Ansprüchen 6 bis 12, wobei sich die Taillenbarriere(n) (5) von 5 % bis 50 %, vorzugsweise von 10 % bis 40 %, noch bevorzugter von 15 % bis 30 %, der gesamten gefalteten Länge (TFL) der äußeren Vliesschicht (10) entlang der Längsachse (y) erstreckt.

14. Saugfähiger Artikel (1) nach einem der vorstehenden Ansprüche, wobei die im Wesentlichen flüssigkeitsundurchlässige Folienschicht oder Rücklage (7) Markierungen, vorzugsweise einen farbigen Aufdruck, an einer Position umfasst, die der quer verlaufenden Taillenbarriere (5) entspricht; und/oder wobei der eine oder die mehreren zweiten elastischen Fäden (12) gefärbt sind; und/oder wobei eine dem Kleidungsstück zugewandte Oberfläche der äußeren Vliesschicht (10) gefärbt ist; und/oder wobei die quer verlaufende Taillenbarriere (5) Markierungen umfasst, die aus einem farbigen Aufdruck und farbigem Klebstoff ausgewählt sind.

15. Saugfähiger Artikel (1) nach einem der vorstehenden Ansprüche, weiter umfassend ein Paar sich längs erstreckender Bündchen, die entlang linker und rechter Längskanten der Einlage (4) positioniert sind, und wobei die quer verlaufende(n) Taillenbarriere(n) (5) oberhalb der Bündchen positioniert ist/sind, sodass die Barriere(n) (5) näher an der Haut eines Trägers ist/sind als die Bündchen; wobei die Bündchen vorzugsweise mit einer dem Körper zugewandten Oberfläche der Oberlage (6) verbunden sind und wobei jedes der Bündchen gefaltet und mit sich selbst auf einer dem Körper zugewandten Oberfläche davon an einer Verbindungsposition (JP) verbunden ist, die im Wesentlichen der/den quer verlaufenden Taillenbarriere(n) (5) entspricht, um eine Exsudatsammeltasche zu bilden.

16. Verfahren zur Herstellung eines saugfähigen Artikels (1), umfassend die Schritte zum:
Bereitstellen einer Vorderseite (2);
Bereitstellen einer Rückseite (3);
Bereitstellen einer saugfähigen Einlage (4), die vordere und hintere Querkanten und linke und rechte Längskanten umfasst, welche die vorderen und hinteren Querkanten verbinden, um einen Umfang davon zu bilden;
Falten eines Abschnitts der saugfähigen Einlage (4) proximal zu den vorderen und/oder hinteren Querkanten davon auf sich selbst, um quer verlaufende Taillenbarriere(n) (5) zu bilden;
Verbinden der Einlage (4) mit der Vorder- und Rückseite (2, 3);
und Verbinden der Vorder- und Rückseite (2, 3) miteinander entlang eines Paares gegenüberliegend angeordneter Seitennähte.

17. Verfahren nach Anspruch 16, weiter umfassend die Schritte des Faltens einer äußeren Vliesschicht (10) der Vorder- und/oder Rückseite (2, 3) über die gefaltete saugfähige Einlage (4) und des Verbindens der gefalteten äußeren Vliesschicht (10) direkt oder indirekt mit der gefalteten saugfähigen Einlage (4).

## Revendications

1. Article absorbant (1) pour l'hygiène personnelle, de préférence une culotte jetable, comprenant :
un panneau avant s'étendant sensiblement transversalement (2) ;
un panneau arrière s'étendant sensiblement transversalement (3) ; et
un insert absorbant s'étendant sensiblement longitudinalement (4) relié à chacun desdits panneaux avant et arrière (2, 3), ledit insert absorbant (4) comprenant des bords transversaux avant et arrière et des bords longitudinaux gauche et droit reliant les bords transversaux avant et arrière pour former un périmètre de celui-ci ;
dans lequel les panneaux avant et arrière (2, 3) sont joints l'un à l'autre pour définir une paire de coutures latérales, et **caractérisé en ce qu'au** moins une partie dudit insert absorbant (4) proximale par rapport aux bords transversaux avant et/ou arrière de celui-ci comprend une barrière de taille transversale (5) comprenant une couche de film sensiblement imperméable aux liquides, et dans lequel ladite au moins une partie dudit insert absorbant (4) proximale aux bords transversaux avant et/ou arrière de celui-ci comprend un ou plusieurs plis formant la ou les barrières de taille transversales (5).

2. Article absorbant (1) selon la revendication 1, dans lequel la couche de film sensiblement imperméable aux liquides comprend une feuille arrière (7), de préférence consistant en celle-ci, de l'insert absorbant (4) et comprend, de préférence, un matériau comprenant un polyester, ou consistant en celui-ci, plus préférentiellement ledit matériau étant choisi dans le groupe consistant en le polyéthylène, l'acide polylactique et le polypropylène.

3. Article absorbant (1) selon l'une quelconque des revendications précédentes, dans lequel ledit pli est sensiblement en forme de U.

4. Article absorbant (1) selon l'une quelconque des revendications précédentes, dans lequel l'insert absorbant (4) comprend une feuille du dessus sensiblement perméable aux liquides (6), une feuille arrière sensiblement imperméable aux liquides (7) et un noyau absorbant (8) pris en sandwich entre celles-ci, de préférence dans lequel le noyau absorbant (8) est positionné à l'intérieur du périmètre dudit insert absorbant (4) lorsqu'il est vu dans une direction plane, de telle sorte que les un ou plusieurs plis soient sensiblement dépourvus de matériau absorbant.

5. Article absorbant (1) selon l'une quelconque des revendications précédentes, dans lequel les panneaux avant et/ou arrière (2, 3) sont élastiques et comprennent une couche non tissée interne (9), une couche non tissée externe (10) et un matériau élastique (11) pris en sandwich entre celles-ci, dans lequel le matériau élastique est choisi dans le groupe consistant en un film élastique et une pluralité de brins élastiques.

6. Article absorbant (1) selon la revendication 5, dans lequel la couche non tissée externe (10) est plus large que la couche non tissée interne (9), généralement dans une direction sensiblement parallèle à l'axe longitudinal y, et est repliée sur ladite couche non tissée interne (9) de telle sorte qu'elle chevauche la partie dudit insert absorbant (4) proximale par rapport aux bords transversaux avant et/ou arrière, de préférence de telle sorte que ladite couche non tissée externe (10) chevauche à la fois une partie de la feuille arrière (7) et une partie de la feuille du dessus (6) qui reste exposée depuis lesdites une ou plusieurs barrières de taille transversales (5) de manière à former un rebord (F_{L}).

7. Article absorbant (1) selon l'une quelconque des revendications précédentes, dans lequel la ou les barrières de taille transversales (5) sont élastiques, comprenant de préférence un matériau élastique choisi dans le groupe consistant en un film élastique et un ou plusieurs brins élastiques.

8. Article absorbant (1) selon les revendications 5 à 7, dans lequel un ou plusieurs seconds brins élastiques (12) sont compris directement ou indirectement entre la couche non tissée externe (10) et la feuille arrière (7), de préférence entre une surface faisant face au corps de la couche non tissée externe (10) et une surface faisant face à un vêtement de la feuille arrière (7).

9. Article absorbant (1) selon l'une quelconque des revendications précédentes, dans lequel le noyau absorbant (8) comprend un matériau absorbant choisi dans le groupe consistant en des particules et/ou des fibres polymères super absorbantes ; et des fibres de cellulose, de préférence dans lequel ledit matériau absorbant est enfermé dans une enveloppe centrale comprenant des couches d'enveloppe centrale supérieure et inférieure qui sont une même couche non tissée pliée pour prendre en sandwich le matériau absorbant en son sein ou des couches non tissées distinctes jointes les unes aux autres pour prendre en sandwich le matériau absorbant en leur sein.

10. Article absorbant (1) selon l'une quelconque des revendications précédentes, dans lequel le panneau arrière (3) est plus large que le panneau avant (2), de telle sorte qu'une longueur des coutures soit sensiblement égale à une largeur du panneau avant (2) et inférieure à une largeur du panneau arrière (3) ; ou dans lequel le panneau avant (2) est plus large que le panneau arrière (3), de telle sorte qu'une longueur des coutures soit sensiblement égale à une largeur du panneau arrière (3) et inférieure à une largeur du panneau avant (2).

11. Article absorbant (1) selon les revendications 2 à 10, dans lequel la feuille arrière (7) comprend une couche de couverture externe supplémentaire (7') sur une surface faisant face à un vêtement de celle-ci, de préférence dans lequel ladite couche de couverture externe (7') comprend une ou plusieurs couches non tissées et/ou dans lequel la feuille arrière (7) comprend une couche de couverture externe supplémentaire(7') qui est plus courte dans la direction longitudinale que la feuille du dessus (6) et/ou la feuille arrière (7), de telle sorte qu'au moins une partie d'une longueur de chevauchement entre l'insert (4) et le panneau (2, 3) soit dépourvue de ladite couche de couverture externe (7'), et de préférence dans lequel la feuille arrière (7) est directement reliée au panneau (2, 3), ou en contact avec celui-ci, dans ladite partie d'une longueur de chevauchement entre l'insert (4) et le panneau (2, 3) dépourvue de ladite couche de couverture externe (7').

12. Article absorbant (1) selon l'une quelconque des revendications précédentes, dans lequel l'insert absorbant (4) est replié pour former la ou les barrières de taille (5), de telle sorte que la feuille du dessus (6) de la partie non pliée dudit l'insert (4) puisse venir en contact avec la partie repliée dudit insert (4).

13. Article absorbant (1) selon les revendications 6 à 12, dans lequel la ou les barrières de taille (5) s'étendent sur 5 % à 50 %, de préférence sur 10 % à 40 %, même plus préférentiellement sur 15 % à 30 %, de la longueur totale pliée (TFL) de la couche non tissée externe (10) le long de l'axe longitudinal (y).

14. Article absorbant (1) selon l'une quelconque des revendications précédentes, dans lequel la couche de film sensiblement imperméable aux liquides, ou la feuille arrière (7), comprend des indices, de préférence une impression colorée, au niveau d'une position correspondant à la barrière de taille transversale (5) ; et/ou dans lequel les un ou plusieurs seconds brins élastiques (12) sont colorés ; et/ou dans lequel une surface faisant face à un vêtement de la couche non tissée externe (10) est colorée ; et/ou dans lequel la barrière de taille transversale (5) comprend des indices sélectionnés parmi une impression colorée et un adhésif coloré.

15. Article absorbant (1) selon l'une quelconque des revendications précédentes, comprenant en outre une paire de revers s'étendant longitudinalement positionnés le long de bords longitudinaux gauche et droit de l'insert (4) et dans lequel la ou les barrières de taille transversales (5) sont positionnées au-dessus desdits revers, de telle sorte que ladite ou lesdites barrières (5) soient plus proches de la peau d'un utilisateur que lesdits revers ; de préférence dans lequel les revers sont reliés à une surface faisant face au corps de la feuille de dessus (6) et dans lequel chacun desdits revers est plié et réuni sur lui-même sur une surface faisant face au corps de celui-ci au niveau d'une position de jonction (JP) correspondant sensiblement à la ou aux barrières de taille transversales (5) pour former une poche de collecte d'exsudat.

16. Procédé de fabrication d'un article absorbant (1), comprenant les étapes consistant à :
fournir un panneau avant (2) ;
fournir un panneau arrière (3) ;
fournir un insert absorbant (4) comprenant des bords transversaux avant et arrière et des bords longitudinaux gauche et droit reliant les bords transversaux avant et arrière pour former un périmètre de celui-ci ;
plier une partie dudit insert absorbant (4) proximale par rapport aux bords transversaux avant et/ou arrière de celui-ci sur elle-même pour former une ou plusieurs barrières de taille transversales (5) ;
joindre ledit insert (4) auxdits panneaux avant et arrière (2, 3) ;
et joindre lesdits panneaux avant et arrière (2, 3) les uns aux autres le long d'une paire de coutures latérales disposées de manière opposée.

17. Procédé selon la revendication 16, comprenant en outre les étapes consistant à plier une couche non tissée externe (10) des panneaux avant et/ou arrière (2, 3) sur l'insert absorbant plié (4) et joindre ladite couche non tissée externe pliée (10) directement ou indirectement à l'insert absorbant plié (4).
